Europäisches Patentamt

(19) European Patent Office

Office européen des brevets

(11) Publication number : **0 445 079 A2**

(12) **EUROPEAN PATENT APPLICATION**

(21) Application number : 91830002.1

(22) Date of filing : 11.01.91

(51) Int. Cl.⁵ : **A61M 1/00, A61M 1/36,**
**A61B 19/00**

The title of the invention has been amended (Guidelines for Examination in the EPO, A-III, 7.3).

(30) Priority : 15.01.90 IT 930790

(43) Date of publication of application :
04.09.91 Bulletin 91/36

(84) Designated Contracting States :
AT BE CH DE DK ES FR GB GR LI NL SE

(71) Applicant : **E.M.S. S.R.L.**
**234, Viale M. Rapisardi**
**Catania (IT)**

(72) Inventor : **Budello, Renzo**
**248, Via Etnea**
**Catania (IT)**

(74) Representative : **Bardini, Marco Luigi et al**
**c/o Società Italiana Brevetti, Corso dei Tintori,**
**25**
**Florence (IT)**

(54) **Apparatus for the perfusion of cardioplegic solutions during heart surgery.**

(57)   An apparatus for the perfusion of cardioplegic solutions and related applications during heart surgery, suitable of handling both crystalloid and sanguineous solutions. The sanguineous type is prepared with a peristaltic pump (11) that features two pairs of rollers. The cardioplegic solution is fed to an heat exchanger (1) where the temperature is regulated to values between 4°C and 37°C by circulating a heat transfer fluid at a controlled temperature. The solution is sucked by a second peristaltic pump (14), passed through a filter (15a) and made available to the surgeon for the perfusion to the patient. Filter means (15), pressure and flow rate control means (19, 23) for the solution are provided. The exchanger (1) is disposable, as also are the various tubes (3, 5) and fittings used in preparing and perfusing the sanguineous solution. With the entire apparatus trolley-mounted and transportable, the perfusionist can prepare and distribute either type of solution without utilizing any auxiliary equipment or accessories.

# APPARATUS FOR THE PERFUSION OF CARDIOPLEGIC SOLUTIONS, AND SIMILAR APPLICATIONS, DURING HEART SURGERY

The invention falls within the art field of heart surgery, and relates more specifically to an apparatus for the perfusion of cardioplegic solutions and for other similar applications, during heart surgery.

In order to carry out an operation on the heart, the organ itself first has to be immobilized by perfusion with a cardioplegic solution. The heart ceases to beat when the temperature of the perfused fluid registers at less than 10°C; conventionally, such liquids are used at 4°C. Once the surgical operation has been accomplished, perfusion of the cardioplegic solution is discontinued, whereupon oxygenated blood is introduced at body temperature to restore the heartbeat.

Two types of cardioplegic solution are adopted in heart surgery:

1 - crystalloid cardioplegic solution;

2 - sanguineous cardioplegic solution, consisting in a crystalloid cardioplegic solution and oxygenated blood together, in a blend of 1 part to 4.

Currently, crystalloid cardioplegic solution is perfused into the patient in one of the following ways: a) a pre-frozen bag of the crystalloid cardioplegic solution is partially defrozen, whereupon the fluid can be administered by applying pressure to the bag;

b) the solution is caused to pass through a coil immersed in a bath of water and ice, or directed by means of one of the free heads of an auxiliary peristaltic circulation pump to an exchanger wherein a coolant from a separate thermostate is circulated;

c) the solution is administered using apparatus equipped with a peristaltic pump by which water from a container filled with water and ice is supplied to an exchanger.

Notwithstanding a great interest in sanguineous cardioplegic solutions, which has been revealed in recent conventions attended by heart surgeons and perfusion technicians, such solutions are notably difficult to prepare and administer, hence their limited adoption.

The only method in current use for administering such a solution is that of inserting two flexible tubes, one of 1/4" diameter (6.35mm) and the other 1/8" (3.2mm), beneath the rollers of a vacant head of the auxiliary peristaltic circulation pump; this is particularly difficult to accomplish, since the pressure required to flatten the 1/8" diameter tube also has the effect of pinching the 1/4" tube. With this pumping arrangement, crystalloid cardioplegic solution is drawn from the respective supply, and blood from the oxygenator. On the outlet side, the two tubes are connected into a 3-way coupling from which the fluids flow united to a small container with an integral exchanger, thence to the patient. Temperature conditioning is achieved either by heat exchange with a liquid supplied from a separate thermostat facility, or by directing the outlet flow from the container through a coil immersed in water and ice prior to reaching the patient.

In none of these instances is there any control of any nature over flow rate, temperature and pressure of the cardioplegic solution. More especially there is no way, using current facilities, of avoiding the thermal shocks to which the heart is subjected prior to and following surgery when passing from normal body temperature of around 37°C down to the 4°C of the solution, then from 4°C back again to the 37°C body temperature of the oxygenated blood which restores heartbeat. With facilities currently available, moreover, one is also confronted with the problem of procuring solution at least in such quantity as is effectively needed for the purpose of heart surgery, which in the case of sanguineous cardioplegic solution entails the use of notable quantities of blood.

The principal object of the present invention is to provide an apparatus for the perfusion of cardioplegic solutions during heart surgery such as will render the task of the perfusionist considerably easier by permitting the use of crystalloid and sanguineous cardioplegic solutions alike, enabling preparation of the solution in real time without any need for prior assessment of the quantity of blood required, and affording control over flow rate, pressure and temperature with the particular end in view of avoiding subjection of the heart to the thermal shock as mentioned above.

A further object of the invention is to provide a peristaltic pump for perfusion apparatus such as will accommodated two tubes of dissimilar diameter at one and the same time without occasioning the difficulties typically encountered hitherto.

The stated objects are realized in apparatus as characterized in the appended claims, designed for the perfusion of cardioplegic solutions and other related applications during heart surgery, and comprising: a heat exchanger in receipt of a transfer fluid and of the cardioplegic solutions, affording inlet, outlet, vent and recycle ports open to the solutions, and a heat exchange surface bathed by the transfer fluid, a first inlet circuit connected to a supply of cardioplegic solution, through which the solution is directed into the exchanger; a second inlet circuit through which a second cardioplegic solution is directed into the exchanger, comprising means for blending the constituents of such a solution; a circuit from which the cardioplegic solutions are recycled to the exchanger and perfused to the patient; means by which to circulate and to monitor and control the temperature and flow rate of the transfer fluid.

In a preferred embodiment of the invention, means for blending the constituents of a cardioplegic solution, and in particular those of sanguineous cardioplegic solution consisting in 1 part of a crystalloid solution and 4 parts blood, comprise a peristaltic pump affording two pairs of rollers by which two tubes, a first carrying the crystalloid solution and a second carrying oxygenated blood measuring twice the diameter of the first, are flattened at one and the same time. To advantage, the various components of the apparatus will be compactly assembled and mounted to a trolley.

Further characteristics and advantages of the apparatus according to the present invention will now be described in detail, by way of a not limiting example, with the aid of the accompanying drawings, in which:

    – fig. 1 is a diagram showing the circuits by means of which cardioplegic solutions are prepared, thermostatically conditioned and perfused to a patient, in an apparatus according to the invention;

    – fig. 2 is a diagram of the circuit for the control of the temperature of the transfer fluid and of the pressure and flow rate of the solution to be perfused, in an apparatus according to the invention;

    – fig. 3 is the side elevation of a peristaltic pump with two pairs of rollers for the apparatus according to the invention;

    – fig. 4 is a top plan view of the peristaltic pump of fig. 3;

    – figs. 5, 6, 7 are elevational views of apparatus according to the invention, viewed in its entirety from the front, side and rear respectively.

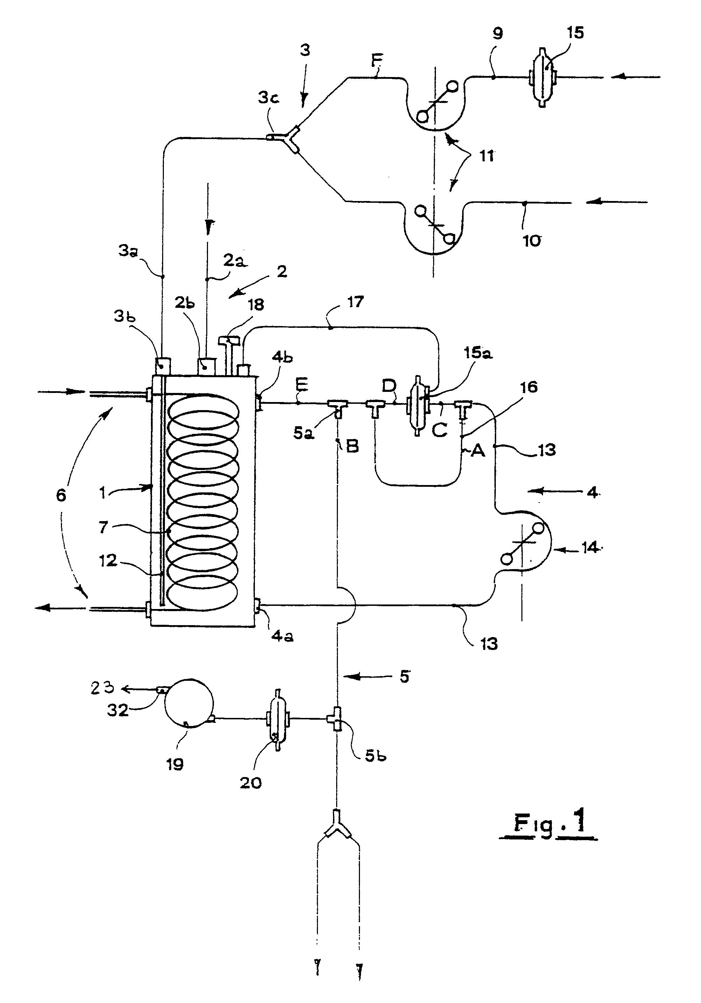

Referring to fig. 1, the apparatus for the perfusion of cardioplegic solutions according to the present invention comprises a heat exchanger 1, of conventional type, connected to a first inlet circuit 2 for a crystalloid cardioplegic solution, a second inlet circuit 3 for the preparation of a sanguineous cardioplegic solution, a recycle circuit 4 through which the solutions are recirculated to the heat exchanger 1, a perfusion circuit 5 to be connected to the patient, and a heat transfer circuit 6 by way of which a heat transfer fluid is circulated into a heat exchange element 7 consisting, for example, of a coil or a finned surface, schematically shown with an helicoid in fig. 1 housed in the heat exchanger 1. The first inlet circuit 2 is connected by way of a relative tube 2a to a bag or container (not illustrated) filled with the crystalloid solution, such that compression of the bag causes the solution to flow through the tube 2a and drop through a relative port 2b into the exchanger 1.

The second inlet or preparation circuit 3 comprises two tubes 9 and 10 connected respectively to a bag of crystalloid solution and to a blood oxygenator (neither supply is illustrated). The two tubes 9 and 10, which are flexible and of dissimilar diameter, the former being half of the latter, namely 1/8" (3.2mm) and 1/4" (6.35mm) respectively, are fed through a peristaltic pump 11 incorporating two pairs of rollers before converging by way of a three-way coupling 3c into a single tube 3a connected to a corresponding port 3b at the top of the exchanger. The sanguineous cardioplegic solution thus obtained consists in the crystalloid solution blended with oxygenated blood at a rate of 1 part to 4. To the end of avoiding red corpuscle trauma that would be occasioned if the solution were to fall freely into the exchanger, flow through the tube 3a is carried beyond the inlet port 3b into a pipe 12 extending dowin to a point near the bottom of the exchanger 1. A filter 15 is installed on the crystalloid solution line 9, upstream of the pump 11.

The recycle circuit 4 comprises a tube 13 connected to an outlet 4a at the bottom of the exchanger 1 and passing beneath the rollers of a peristaltic pump 14 by which the solution is both perfused to the patient and returned by way of another inlet 4b to the exchanger 1. The section of a circuit 4 downstream of the pump 14 comprises a filter 15a through which crystalloid solutions are passed, a filter by-pass loop 16, and a T-joint 5a from which the perfusion circuit 5 is taken off. The filter 15a and exchanger 1 are interconnected additionally by a tube 17 for removing air bubbles from the crystalloid solution, the exchanger 1 being fitted uppermost with a valve 18 through which to vent any such air. A pressure gauge 19, and a non-return and antibacterical filter 20 are installed on the perfusion circuit 5 in correspondance to a respective T-joint 5b. All the tubes thus mentioned are embodied in flexible and elastic material, and can therefore be locked at given points to stop the flow of solution, and flattened by the rollers of the peristaltic pumps 11 and 14. In particular, the lenght of tube interconnecting the two T-joints 5a and 5b of the perfusion circuit 5 will be elastically deformable to a high degree to the end of absorbing fluctuations in pressure and flow rate due to lack of absolute uniformity in the action of the peristaltic pump 14.

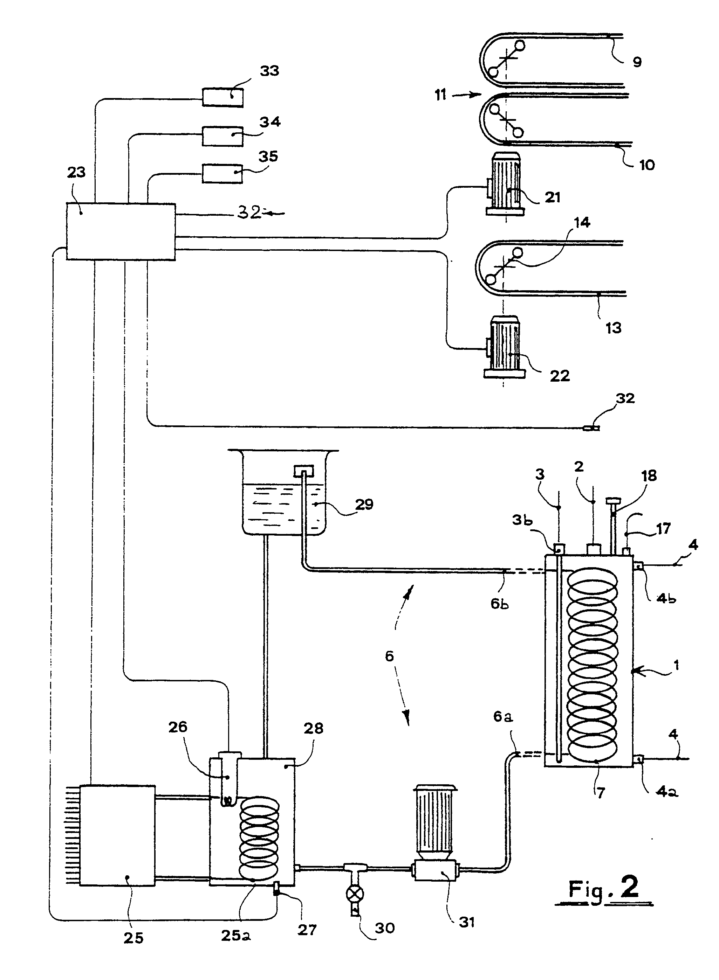

Referring to fig. 2, the peristaltic pumps 11 and 14 are driven by respective geared motors 21 and 22 interlocked to an electronic monitoring and control circuit 23 of conventional type; this circuit also controls the temperature of the transfer fluid circulating within the heat transfer circuit 6 and through the coil 7 internally of the exchanger 1.

The heat transfer circuit 6 comprises a thermostatic vessel 28 accommodating the immersed coil 25a of a refrigeration unit 25, together with an electrical resistance 26 and a thermal probe 27 wired to the control circuit 23; accordingly, the refrigeration unit 25 and the resistance 26 are governed by the electronic circuit 23 in such a way as to hold the temperature of the transfer fluid at a desired value.

The circuit 6 further comprises an expansion vessel 29 installed on the inlet side of the thermostatic vessel 28 and serving to replenish the circuit 6, a drain cock 30, and a pump 31 installed downstream of the vessel 28 by which the fluid is circulated. The electronic monitoring and control circuit 23 also comprises a pressure transducer 32 (see fig. 1) connected to the pressure gauge 19, and digital displays 33, 34 and 35 respectively indicating the values of flow rate from the peristaltic perfusion pump 14, the temperature of the transfer fluid as sensed by the thermal probe 27, and the perfusion pressure registering at the gauge 19. In an alternative embodiment, the thermostatic vessel 28 might incorporate thermoelectric modules (Peltier elements) in place of the coil 25a of the refrigeration unit 25 and the resistance 26.

These conventional devices operate by absorbing or giving off heat according to the direction of a current passed through them; thus, appropriately piloted, each such module can operate both as a cooling and as a heating medium.

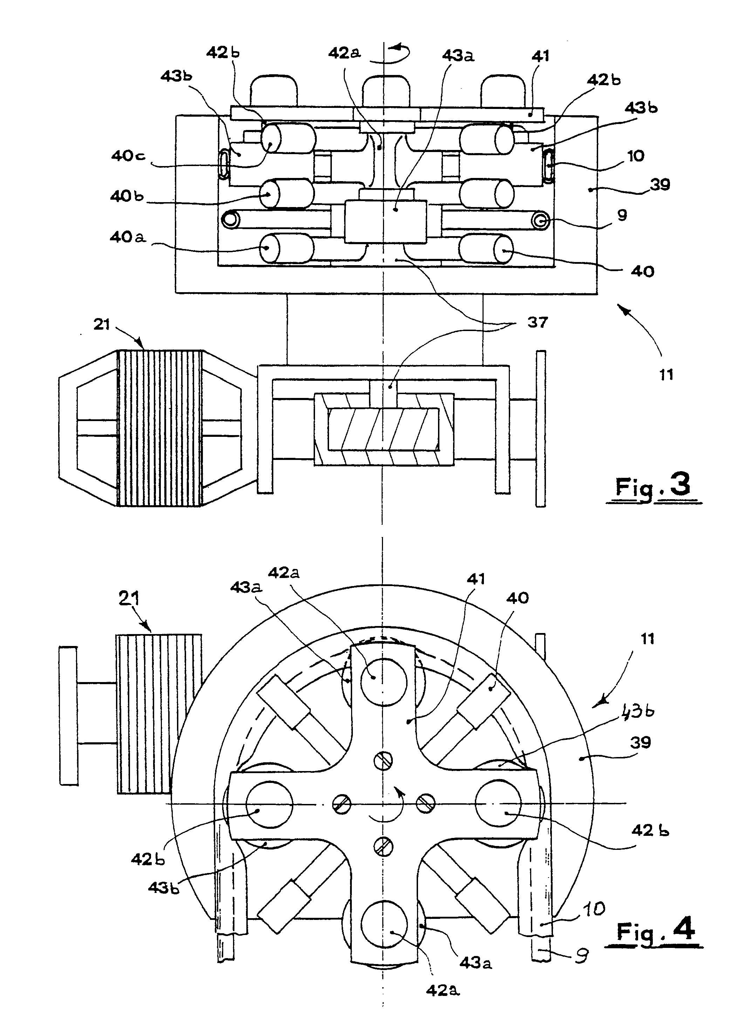

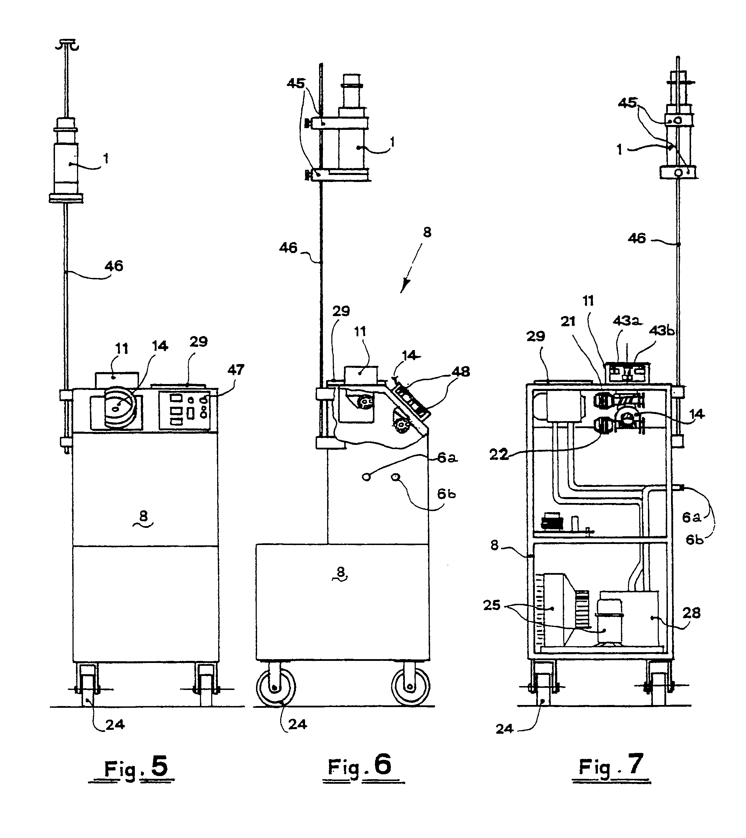

With reference now to figs 3 and 4, the peristaltic pump 11 fitted with two pairs of rollers comprises a shaft 37 set in rotation by the relative geared motor 21. The shaft 37 is supported rotatably in a substantially semicylindrical fixed housing 39, and carries three perpendicular pairs of keyed tube guide elements 40 occupying planes disposed mutually equidistant and parallel, normal to the axis of the shaft 37, and a cross support 41. Each of the four arms of the cross support 41 carries a fixed pivot 42 and a roller 43 mounted rotatably to the end of the pivot 42. More particularly, the two pivots 42a of one pair of aligned arms of support 41 are of lenght greater than that of the pivots 42b of the remaining pair, such that the rollers 43a carried by the first two pivots 42a occupy a median plane between the planes of a first two tube guide elements 40a and 40b (starting from the bottom of housing 39), whereas the two rollers 43b carried by the remaining pivots 42b occupy a median plane between the planes occupied by a second two guide elements 40b and 40c.

As illustrated in figs 3 and 4, the tube 9 carrying the crystalloid solution is flattened by the first pair of rollers 43a against the inside wall of the semicylindrical housing 39, positioned between the two guide elements 40a and 40b, whilst the tube 10 carrying oxigenated blood (measuring twice the diameter of the solution tube 9) is flattened by the second pair of rollers 43b, guided between the two elements 40b and 40c.

Referring to figs. 5, 6 and 7, the entire assembly of components making up the circuits described above can be mounted to a single trolley 8 running on conventional wheels 24, by which the apparatus is thus supported and transported. In particular, the exchanger 1 is mounted to a bracket 45 adjustable vertically on a standard 46 associated rigidly with the trolley 8, and connected to the heat transfer circuit 6 by way of tubes (not shown) coupled to corresponding flow and return ports 6a and 6b located at the side of the trolley 8. The two supply tubes 9 and 10 and the recycle tube 13 (not shown in figs. 5, 6, and 7) are connected to the exchanger 1 by the perfusionist as described previously, and engaged with the rollers 43a-43b and 48 of the respective peristaltic pumps 11 and 14.

The trolley 8 will also incorporate an electronic console 47, connected to the monitoring and control circuit 23, from which the operator can oversee the various functions of apparatus according to the invention.

To effect a perfusion of crystalloid cardioplegic solution with the circuits arranged as in fig 1, a bag of the solution must first be connected to the relative inlet tube 2a and the exchanger 1 filled in part; this done, the peristaltic pump 14 must be activated after having engaged the recycle tube 13 therewith, and then the circuit 4 is locked at point A, and at point B also, initially. The circuit is even locked at point D in order to direct the solution into the filter vent line 17 and release any air bubbles. Once the right conditions have been established, the solution will pass through the filter 15a and back to the exchanger 1 by way of the return port 4b, whereupon locking at E and releasing at B, the solution can be made available to the surgeon for perfusion through the organ.

To effect perfusion with a sanguineous cardioplegic solution, on the other hand, the tubes 9 and 10 from the crystalloid solution and oxygenated blood supplies ( not illustrated) must be positioned under the twin rollers of the double peristaltic pump 11 as in figs 3 and 4, and the pump set in motion.

Accordingly, the cardioplegic solution entering the exchanger 1 by way of the filler pipe 12 is a 1:4 blend of crystalloid solution and oxygenated blood. Given that the sanguineous solution cannot pass through the filter 15a, the recycle circuit 4 must be locked at C and remain open at A so as to direct flow through the by-pass loop 16. The rest of the procedure remains the same as described above for crystalloid solutions.

While the above steps are in progress, a check is kept on the temperature of the fluid occupying the thermostatic vessel 28 (between 4°C and 37°C), which will be circulated by the pump 31 through the coil 7 of the heat exchanger 1 (see fig. 2). By way of example, a transfer fluid flow rate of 8 l/min coupled with a heat exchange surface of 2 square metres will be sufficient to lower the temperature of the cardioplegic solution in the exchanger 1 to the desired temperature, generally from 37°C down to 4°C, within a few seconds.

With surgery terminated, the twin roller pump 11 used to prepare the sanguineous solution can also be utilized to perfuse oxygenated blood alone by locking off the crystalloid solution supply tube 9 at F (fig. 1). In this situation, the temperature of the oxygenated blood is raised thermostatically to the requisite value, increasing gradually from 4°C to 37°C internally of the exchanger 1, whereupon the blood is drawn from the bottom port 4b of the exchanger by the peristaltic recycle pump 14 and made available to the surgeon by the same route as that employed in cardioplegia, in this instance for the purpose of reviving the heart of the patient.

Using the apparatus according to the present invention, the perfusionist needs no additional systems or accessories in order to effect either one of the two types of cardioplegic perfusion. A sanguineous solution can be prepared without any difficulty, inasmuch as the peristaltic blending pump 11 is able to deform supply tubes of dissimilar diameter simultaneously; whilst in the example disclosed the two diameters are 1/8″ (3.2mm) and 1/4″ (6.35mm), other tubes of equivalent relative proportions might equally well be utilized. Thanks to the high efficiency of the exchanger 1, the solutions to be perfused can be prepared substantially in real time and without any advance provision being made in respect of quantities; accordingly, there is no need to keep a large stock of patient's blood on hand. The exchanger 1 also permits of using the solution down to the last 20 millilitres, as with the perfusion pump 14 drawing from a bottom port 4b, practically the entire contents of the enclosure can be drawn off without any fear of including air bubbles. In effect, with a valve 18 installed at the top of the exchanger 1, any air bubbles that may form in the circuit can be vented continuously. In conventional apparatuses, by contrast, the perfusionist is obliged to open up and retighten the valve 18 continually in maintaining a level of pressure sufficient to ensure that the liquid reaches the patient. In the apparatus according to the invention, moreover, pressure, flow rate and temperature of the liquids can be monitored and adjusted swiftly to suit the requirements and techniques of the individual heart surgeon. Considered in its entirety and from the standpoint of components parts (peristaltic pumps, thermostatic monitoring and control systems etc.), cardioplegic perfusion apparatus according to the invention will be seen to afford a notable degree of flexibility as regards adaptability to such other uses as may be required by the surgeon.

The exchanger 1 is disposable, as well as the circuits between the exchanger and the remainder of the equipment and between the exchanger and the patient, thereby eliminating problems of infection and sterilization.

With apparatus that is compact in embodiment and readily transportable when mounted to a trolley 8, one achieves a saving in space that takes on great importance in the operating room.

Variations and/or modifications may be brought to the apparatus for the perfusion of cardioplegic solutions and similar applications during heart surgery, according to the present invention without departing from the scope of the invention itself.

## Claims

1. Apparatus for the perfusion of cardioplegic solutions and similar applications during heart surgery, characterized in that it comprises:
   – heat exchange means in receipt of a heat transfer fluid and of said cardioplegic solutions, comprising an exchanger (1) affording inlet, outlet, vent and recycle ports (2b, 3b, 4a, 18, 4b) for said solutions, and a heat exchange element (7) in which said heat transfer fluid flows;
   – a first inlet circuit (2) through which a first cardioplegic solution is fed from a relative supply into the exchanger (1);
   – a second inlet circuit (3) through which a second, blended cardioplegic solution is fed into the exchanger (1), comprising means for blending the constituents of said solution;
   – a recycle and perfusion circuit (4, 5) connected to said heat exchange means by way of said outlet and recycle ports (4a, 4b), from which the cardioplegic solutions are returned to the exchanger and perfused to the patient;
   – means for circulating (6) said heat transfer fluid fed to said exchanger (1) and for controlling the temperature and flow rate thereof.

2. Apparatus according to claim 1, wherein said blending means of the second inlet circuit (3) comprise a first peristaltic pump (11) incorporating two pairs of rollers (43a, 43b) for flattening two tubes (9, 10) of dissimilar section, coming from a supply of crystalloid solution and from a blood oxygenator respectively, said tubes converging downstream of the pump into an inlet (3a) of said exchanger (1).

3. Apparatus according to claim 2, wherein said peristaltic pump (11) comprises a shaft (37) set in rotation by drive means (21), a fixed housing (39) affording an internal circular wall concentric with said shaft, a support (41) rigidly associated with said shaft and carrying two pairs of rollers (43a, 43b) occupying two distinct planes for flattening against said wall two flexible tubes (9, 10) arranged circumferentially along said wall, tube guide (40) means radially extending from said shaft (37) being provided for keeping said tubes spaced apart at unvarying distance.

4. Apparatus according to claim 3, wherein said support (41) comprises four pivots (42a, 42b) projecting from one face of said support, positioned at the same distance from the axis of rotation of said shaft (37), spaced apart at 90° from one another, at the ends of which said rollers (43a, 43b) are pivotally connected, each of said pivots being equal in lenght to the pivot diametrically opposite and dissimilar in lenght to the pivot adjacent on either side.

5. Apparatus according to claim 4, wherein said support (41) carrying said rollers (43a, 43b) comprises four identical arms arranged crosswise, at the end of each of them the pivots (42a, 42b) being rigidly associated.

6. Apparatus according to the claims 3, 4 and 5 wherein said tube guide means (40) comprises three pairs of radially extending crossed arms (40a, 40b, 40c) occupying planes disposed mutually parallel and equidistant, normal to the axis of the shaft (37), of which the ends are located a short distance from the internal circular wall of the housing (39).

7. Apparatus according to the preceeding claims, wherein circulation means (6) comprises a thermostatic vessel (28) filled with said fluid, and cooling means (25), heating means (26) and thermal sensing means (27) immersed therein for the regulation and the control of the temperature of said fluid.

8. Apparatus according to claim 7, wherein said cooling means (25) and heating means (26) immersed in the heat transfer fluid comprises respectively a refrigeration coil (25a) and an electrical resistance (26 ).

9. Apparatus according to claim 7, wherein said cooling means and heating means immersed in the transfer fluid comprise thermoelectric Peltier elements.

10. Apparatus according to the previous claims, wherein the recycle and perfusion circuit (4, 5) from which cardioplegic solutions are returned to the exchanger (1) and perfused to the patient comprises a second peristaltic pump (14), filter means (15a) for filtering the solution, vent means (17) for removing air bubbles from the solution, and pressure sensing means (19) for monitoring the pressure of the solution, the perfusion circuit (5) comprising substantially a lenght ot elastically deformable tube capable of absorbing fluctuations in pressure and flow rate due to the second peristaltic pump (14).

11. Apparatus according to the previous claims, further comprising a trolley (8) for supporting and transporting the various means and circuits, wherein said exchanger (1) is carried by a bracket (45) supported slidably in the vertical direction on a standard (46) associated rigidly with said trolley (8), said trolley (8) incorporating an electronic monitoring and control console (47).

12. A peristaltic pump comprising two pairs of rollers, as recited in claims 2, 3, 4 and 5.

Fig.1

**Fig. 2**

Fig. 3

Fig. 4

Fig.5

Fig.6

Fig.7